# EUROPÄISCHE PATENTSCHRIFT

(11) **EP 1 389 963 B1**
(45) Veröffentlichungstag und Bekanntmachung des Hinweises auf die Patenterteilung: **14.06.2006**
(21) Anmeldenummer: 01931311.3
(22) Anmeldetag: 28.05.2001
(51) Int. Cl.: A61B 17/80

(54) **KNOCHENPLATTE ZUR FIXATION VON PROXIMALEN HUMERUSFRAKTUREN**
BONE PLATE FOR THE FIXATION OF FRACTURES OF THE PROXIMAL HUMERUS
LAME OSSEUSE POUR LA FIXATION DE FRACTURES DE L'HUMERUS PROXIMAL

(43) Veröffentlichungstag der Anmeldung: 25.02.2004
(73) Patentinhaber: Synthes AG Chur, 7002 Chur (CH)
(72) Erfinder: NIEDERBERGER, Alfred, CH-2540 Grenchen (CH); HATTLER, Eric, CH-4500 Solothurn (CH)
(74) Vertreter: Lusuardi, Werther
(86) Internationale Anmeldenummer: PCT/CH2001/000327
(87) Internationale Veröffentlichungsnummer: WO 2002/096309

(56) Entgegenhaltungen:
- WO-A-00/53110
- WO-A-01/19267
- US-A- 5 197 966
- US-A- 5 938 664
- US-A- 6 096 040

## Beschreibung

Die Erfindung betrifft eine Knochenplatte zur Fixation von proximalen Humerusfrakturen gemäss dem Oberbegriff des Patentanspruchs 1.

Solche Knochenplatten dient zur Fixation von allen Frakturtypen am proximalen Humerus, jedoch insbesondere für alle drei- und vier-part Frakturen sowie für dislozierte Frakturen am proximalen Humerus.

Eine häufige Ursache der Frakturen des proximalen Humerus in jungen Jahren sind Hochenergie-Traumata. Diese Frakturart nimmt in zunehmendem Alter sogar zu, da sich im Bereich des proximalen Humerus die Knochenstruktur so sehr verschlechtert, so dass nur noch in der Randzone des Knochens intakte Knochenstruktur vorhanden ist. Bei einem Sturz auf den ausgestreckten Arm im fortgeschrittenen Alter bricht der Knochen dann vorzugsweise an dieser Stelle.

Aus der US-A 6,096,040 ESSER ist bereits eine gattungsgemässe Knochenplatte bekannt.

Die Nachteile dieser Anordnung bestehen darin, dass die Platte im löffelartigen Teil eine Lochposition aufweist, mit welcher es nicht möglich ist, die bekanntermassen gute Knochenstruktur in der Randzone des proximalen Humerus zu fassen.
Zudem handelt es sich bei dem bei ESSER beschriebenen Implantat, um eine Platte ohne winkelstabile Optionen in den Plattenlöchern. Bei der Verwendung der Platte wird diese mit Hilfe von Knochenschrauben an den Knochen gepresst. Dieses Vorgehen nennt man Kompressionsosteosynthese. Dabei werden die auftretenden Kräfte über die Reibung zwischen Implantat und Knochen übertragen, während der Knochen einen Grossteil der Last tragen muss. Durch den grossen axialen Druck, welchem die Knochenschrauben für eine relativ, stabile Sicherung der Platte an den Knochen ausgesetzt sind, kommt es unter dynamischen Bedingungen zu einem Ausriss der Knochenschrauben aus dem Knochen und damit einhergehend einem Verlust der Stabilität des Platten-Knochenkonstrukts..

Die obenstehende Diskussion des Standes der Technik erfolgt lediglich zur Erläuterung des Umfeldes der Erfindung und bedeutet nicht, dass der zitierte Stand der Technik zum Zeitpunkt dieser Anmeldung oder ihrer Priorität auch tatsächlich publiziert oder öffentlich bekannt war.

Hier will die Erfindung Abhilfe schaffen. Der Erfindung liegt das Problem zugrunde, eine Knochenplatte zur Fixation von proximalen Humerusfrakturen zu schaffen, mit welcher eine winkelstabile Osteosynthese erreicht werden kann. Wesentliche Vorteile der Erfindung sind die Winkelstabilität der Schraubenlöcher in Kombination mit Schrauben mit Kopfgewinde, die einfache Handhabung, die optimalere anatomische Anpassung an den Knochen und keine Behinderung der relevanten anatomischen Strukturen des proximalen Humerus. Durch die Verwendung von Schrauben mit Gewindekopf entsteht ein winkelstabiles Platten-Knochenkonstrukt, welches den Halt auch unter dynamischen Bedingungen zu bewahren vermag.

WO01/19267 offenbart ein Knochenplatte mit der Merkmale im Oberbegriff des Anspruchs 1. Die Erfindung löst die gestellte Aufgabe mit einer Knochenplatte, welche die Merkmale des Anspruchs 1 aufweist.

Die geringe Gewindesteigung erlaubt auf einer sehr kurzen Strecke, üblicherweise im Bereich von 1 bis 2 mm, eine feste Verankerung der Schraube in der Platte. Dadurch kann die Dicke der Platte sehr dünn dimensioniert werden, ohne dass eine Beeinträchtigung der Stabilität des Platten-Schrauben-Konstrukts die Folge wäre.

Eine bevorzugte Weiterbildung besteht darin, dass die Steigung der Innengewinde oder der partiell umlaufenden Keilnuten im Bereich von 0,7 bis 0,9 mm liegt, was den oben beschriebenen Effekt weiter optimiert.

Bei einer weiteren bevorzugten Ausführungsform verbreitert sich der Übergangsbereich von der Breite x des stilförmigen Teils zur Breite y des löffelartigen Teils, vorzugsweise in exponentieller Weise.
Damit lässt sich eine optimale Anpassung an den ungebrochenen proximalen Humerus des Menschen erzielen. Die Platte liegt dadurch sauber am Knochen auf und keine der relevanten anatomischen Strukturen werden behindert. s

Die Dicke der Platte. liegt zweckmässigerweise im Bereich von 1,7 bis 2,3 mm, vorzugsweise zwischen 1,9 bis 2,1 mm. Der Vorteil der gegenüber konventionellen Knochenplatten vergleichsweise geringen Dicke besteht hauptsächlich darin, dass dünnere Platten keine der relevanten Strukturen behindern und beim Heben des Armes nicht am Acromion der Schulter anecken.

Vorzugsweise ist der stilförmige Teil der Platte, in Richtung der Zentralachse gesehen, mindestens in einem Teilbereich gekrümmt ausgebildet .
Bei einer weiteren bevorzugten Ausführungsform ist der stilförmige Teil der Platte, in Richtung der Zentralachse (3) gesehen, mindestens in einem Teilbereich in Form eines Ellipsenbogens (21) gekrümmt. Die Länge der grossen Achse (22) der zum Ellipsenbogen (21) gehörige Ellipse liegt vorzugsweise im Bereich zwischen 150 und 170 mm, typischerweise im Bereich von 157 und 163 mm. Die Länge der kleinen Achse der zum Ellipsenbogen gehörigen Ellipse liegt vorzugsweise im Bereich zwischen 60 und 80 mm, typischerweise im Bereich zwischen 67 und 73 mm.
Das freie Ende des stilförmigen Teils und des löffelartigen Teils (2) liegen vorzugsweise im wesentlichen in zueinander parallelen Ebenen.

All diese bevorzugten Massnahmen haben den Vorteil, dass eine verbesserte Anpassung an den ungebrochenen proximalen Humerus des Menschen möglich ist, wobei die Platte sauber am Knochen aufliegt und keine der relevanten anatomischen Strukturen behindert werden.

Bei einer weiteren bevorzugten Ausführungsform weist die Knochenplatte mindestens in einem Teilbereich eine quer zur Zentralachse verlaufende Krümmung auf, vorzugsweise mit einem Krümmungsradius im Bereich von 18 bis 22 mm.

Bei einer weiteren Ausführungsform weist die Knochenplatte über ihre gesamte Länge eine quer zur Zentralachse verlaufende Krümmung auf. Der damit erzielbare Vorteil liegt darin, dass keine Irritation der vorhandene Weichteile auftritt. Beim Heben des Armes wird verhindert, dass die Knochenplatte am Acromion der Schulter aneckt.

Bei einer weiteren bevorzugten Ausführungsform weist die freie, endständige Partie des löffelartigen Teils zwei symmetrisch zur Zentralachse angeordneten Bohrungen mit parallelen Bohrungsachsen auf, wobei die Bohrungsachsen einen Winkel von 92° bis 98°, vorzugsweise von 94° bis 96° mit der durch den löffelartigen Teil gebildeten Fläche bilden. Um eine winkelstabile Reposition zu erlangen sind bei Implantaten gemäss dem Stand der Technik Klingen erforderlich, welche durch die in den winkelstabile Bohrungen einzuführende Schrauben ersetzt werden. Die Operationstechnik von Klingen erfordert aber einen höheren Zeitaufwand und ist komplexer anzuwenden als jene mit winkelstabilen Schrauben wie bei der Erfindung. Durch den Berührungswinkel von ca. 95° wird die Berührungsfläche mit dem festen Knochen vergrössert, da festes Knochenmaterial nur in den Randzonen des relativ hohlen Humeruskopf vorhanden ist.

Bei einer weiteren bevorzugten Ausführungsform weist die an den Übergangsbereich angrenzende Partie des löffelartigen Teils zwei asymmetrisch zur Zentralachse angeordnete Bohrungen auf und die Bohrungsachsen der beiden Bohrungen liegen in zueinander parallelen Ebenen, welche vorzugsweise orthogonal zur Zentralachse stehen. Die Projektion der einen Bohrungsachse in die parallele Ebene, welche die andere Bohrungsachse enthält, schliesst mit der Bohrungsachse einen Winkel von 40°- 60°, vorzugsweise von 46°- 54° untereinander ein.
Der Vorteil dieser Einrichtung liegt darin, dass die in diese sich schränkenden Bohrungen einzuführenden Schrauben ein Heranziehen des kleinen Tuberkulum erlaubt, welches bei Frakturen des proximalen Humerus häufig abbricht.

Eine weitere bevorzugte Ausführungsform besteht darin, dass mindestens eine der symmetrisch und/oder asymmetrisch zur Zentralachse angeordneten Bohrungen ein Innengewinde oder partiell umlaufende Keilnuten aufweisen. Dabei sollten sich vorteilhafterweise keine der Bohrungsachsen der symmetrisch und/oder asymmetrisch zur Zentralachse angeordnete Bohrungen schneiden. Dadurch wird der Vorteil erzielt, dass sich die in die Bohrungen einzuführende Schrauben nicht gegenseitig behindern.

Bei einer weiteren Ausführungsform stehen die Achsen der im stilförmigen Teil angebrachten Bohrungen senkrecht dazu.

Die Innengewinde sind vorzugsweise mehrgängig, typischerweise zweigängig ausgebildet.

Die erfindungsgemässe Knochenplatte kann sowohl für den linken wie auch für den rechten Humerus verwendet werden und passt sich optimal an die Form des gesunden proximalen Humerus an.

Die Erfindung und Weiterbildung der Erfindung wird im folgenden anhand der teilweise schematischen Darstellung eines Ausführungsbeispiels noch näher erläutert.

Es zeigen:
Fig. 1 eine Aufsicht auf die erfindungsgemässe Knochenplatte;
Fig. 2 einen Schnitt längs der Linie II -II in Fig.1;
Fig. 3 eine perspektivische Darstellung der erfindungsgemässe Knochenplatte; und
Fig. 4 eine Seitenansicht der erfindungsgemässe Knochenplatte.

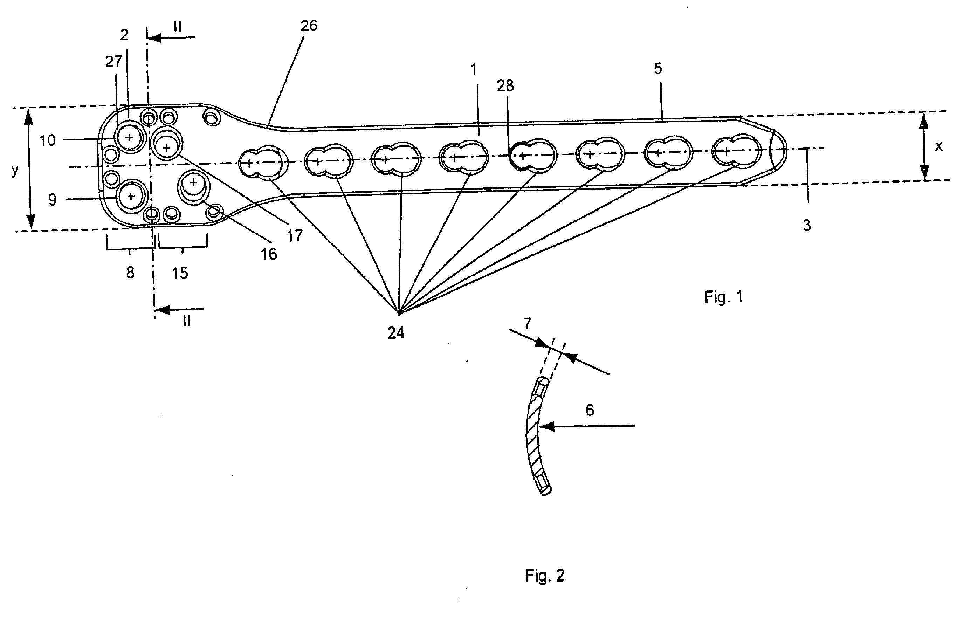

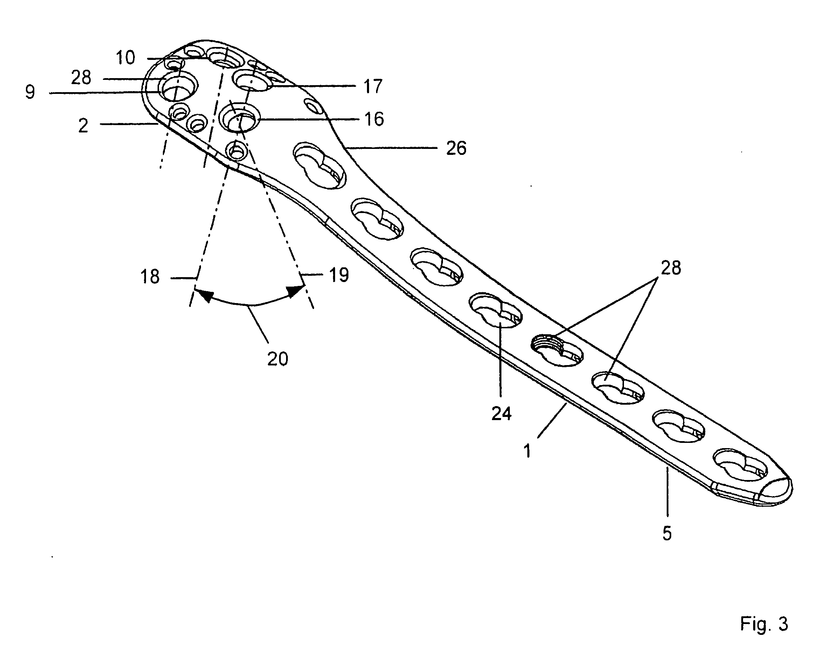

Die in den Fig. 1 bis 4 dargestellte Knochenplatte, welche zur Fixation von proximalen Humerusfrakturen dient, besteht aus einem stilförmigen Teil 1, dessen Länge grösser als seine Breite x ist und einem löffelartigen Teil 2, dessen Breite y grösser als die Breite x des stilförmigen Teils 1 ist. Der stilförmige Teil 1 und der löffelartige Teil 2 weisen eine gemeinsame longitudinale Zentralachse 3 auf und sind durch einen Übergangsbereich 26 miteinander verbunden, der sich von der Breite x des stilförmigen Teils 1 zur Breite y des löffelartigen Teils 2 exponentiell verbreitert.
Im stilförmigen Teil 1 wie auch im löffelartigen Teil 2 sind eine grössere Anzahl von Schraubenlöchern (9,10,16,17;24) angebracht. Alle im stilförmigen Teil 1 der Knochenplatte angebrachten Schraubenlöcher 24 weisen ein Innengewinde 28 auf (dieses könnte auch durch partiell umlaufende Keilnuten ersetzt sein.
Vier der im löffelartigen Teil 2 der Knochenplatte angebrachten Schraubenlöcher (9,10,16,17) weisen ebenfalls ein Innengewinde 27 auf (auch hier könnte das Innengewinde durch partiell umlaufende Keilnuten ersetzt sein.
Die Steigung dieser Innengewinde 27;28 (oder der partiell umlaufenden Keilnuten) beträgt 0,8 mm. Die Dicke der Knochenplatte beträgt 2 mm.

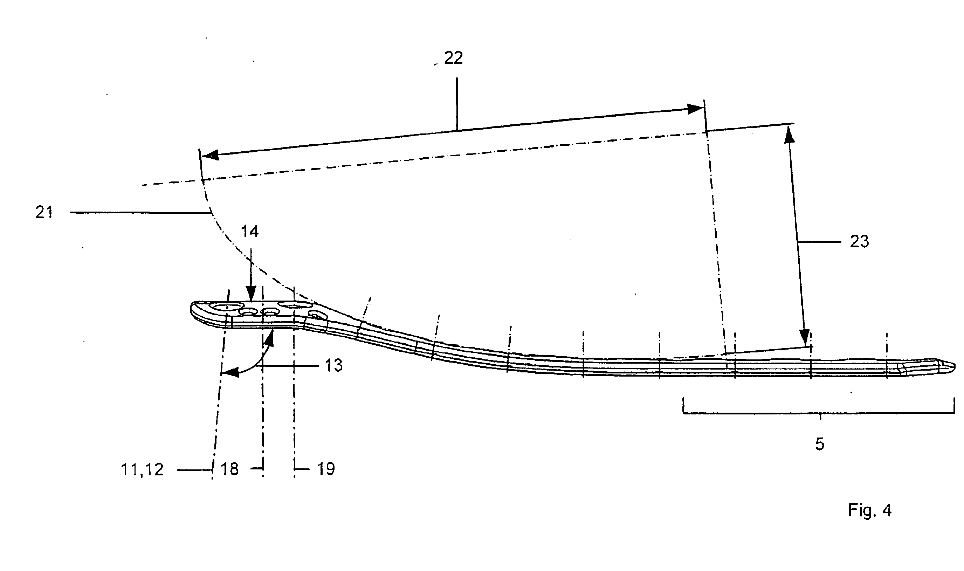

Wie aus Fig. 4 ersichtlich ist der stilförmige Teil 1, in Richtung der Zentralachse 3 gesehen, in einem Teilbereich gekrümmt ausgebildet und zwar in Form eines Ellipsenbogens 21. Die Länge der grossen Achse 22 der zum Ellipsenbogen 21 gehörigen Ellipse beträgt 160 und die Länge der kleinen Achse 23 der zum Ellipsenbogen 2) gehörigen Ellipse beträgt 70 mm. Durch die Krümmung des stilförmigen Teils 1 liegt das freie Ende 5 des stilförmigen Teils 1 und des löffelartigen Teils 2 im wesentlichen in zueinander parallelen Ebenen.

Wie aus Fig. 2 ersichtlich weist die Knochenplatte über ihre gesamte Länge eine quer zur Zentralachse 3 verlaufende Krümmung 6 auf mit einem Krümmungsradius von 20 mm.

Wie aus den Fig. 1 und 2 ersichtlich weist die freie, endständige Partie 8 des löffelartigen Teils 2 zwei symmetrisch zur Zentralachse 3 angeordneten Bohrungen 9,10 mit parallelen Bohrungsachsen auf, wobei die Bohrungsachsen 11,12 einen Winkel 13 von 95° mit der durch den löffelartigen Teil 2 gebildeten Fläche 14 bilden.

Die an den Übergangsbereich 26 angrenzende Partie 15 des löffelartigen Teils 2 weist zudem zwei asymmetrisch zur Zentralachse 3 angeordnete Bohrungen 16, 17 auf. Die Bohrungsachsen 18,19 der beiden Bohrungen 16,17 liegen in zueinander parallelen Ebenen, welche orthogonal zur Zentralachse 3 stehen. Die Projektion der einen Bohrungsachse 18 in die parallele Ebene, welche die andere Bohrungsachse 19 enthält, schliesst mit der Bohrungsachse 19 einen Winkel 20 von 50° ein.

Die symmetrisch und asymmetrisch zur Zentralachse 3 angeordneten Bohrungspaare 9,10,16,17 weisen ein Innengewinde 27 auf, welches auch durch partiell umlaufende Keilnuten ersetzt sein könnte. Keine der Bohrungsachsen 11,12;18,19 der symmetrisch und asymmetrisch zur Zentralachse 3 angeordneten Bohrungspaare 9,10;16,17 schneiden sich. Die Innengewinde 27;28 sind zweigängig ausgebildet.

Die Achsen der im stilförmigen Teil 1 angebrachten Bohrungen 24 stehen senkrecht zum stilförmigen Teil 1.

## Patentansprüche

1. Knochenplatte zur Fixation von proximalen Humerusfrakturen mit einem stliförmigen Teil (1), dessen Länge grösser als seine Breite x ist und einem löffelartigen Teil (2), dessen Breite y grösser als die Breite x des stilförmigen Teils (1) ist, wobei der stilförmige Teil (1) und der löffelartige Teil (2) eine gemeinsame longitudinale Zentralachse (3) aufweisen und durch einen Übergangsbereich (26) miteinander verbunden sind, und wobei im stilförmigen Teil (1) wie auch im löffelartigen Teil (2) mindestens je 2 Schraubenlöcher (9;10,16;17;24) angebracht sind, wobei
mindestens eines der Schraubenlöcher (24) im stitförmigen Teil (1) ein mindestens partielles Innengewinde (28) oder partiell umlaufende Keilnuten aufweist; und
mindestens eines der Schraubenlöcher (9,10,16.17) im löffelartigen Teil (2) ein Innengewinde (27) oder partiell umlaufende Keilnuten aufweist; **dadurch gekennzeichnet, dass**
die Steigung der Innengewinde (27;28) oder der partiell umlaufenden Keilnuten im Bereich von 0,5 mm bis 1,1 mm liegt; und
die Dicke (7) der Knochenplatte im Bereich von 1,7 bis 2,3 mm liegt

2. Knochenplatte nach Anspruch 1, **dadurch gekennzeichnet, dass** die Steigung der Innengewinde (27;28) oder der partiell umlaufenden Keilnuten im Bereich von 0,7 bis 0,9 mm liegt.

3. Knochenplatte nach Anspruch 1 oder 2, **dadurch gekennzeichnet, dass** sich die Breite des Übergangsbereiches (26) von der Breite x des stilförmigen Teils (1) zur Breite y des löffelartigen Teils (2) verbreitert, vorzugsweise in exponentieller Weise.

4. Knochenplatte nach einem der Ansprüche 1 bis 3 , **dadurch gekennzeichnet, dass** ihre Dicke (7) im Bereich von 1,9 bis 2.1mm liegt.

5. Knochenplatte nach einem der Ansprüche 1 bis 4, **dadurch gekennzeichnet, dass** der stilförmige Teil (1), in Richtung der Zentralachse (3) gesehen, mindestens in einem Teilbereich gekrümmt ausgebildet ist.

6. Knochenplatte nach einem der Ansprüche 1 bis 5, **dadurch gekennzeichnet, dass** der stilförmige Teil (1), in Richtung der Zentralachse (3) gesehen, mindestens in einem Teilbereich in Form eines Ellipsenbogens (21) gekrümmt ist.

7. Knochenplatte nach Anspruch 6, **dadurch gekennzeichnet, dass** die Länge der grossen Achse (22) der zum Ellipsenbogen (21) gehörige Ellipse im Bereich zwischen 150 und 170 mm, vorzugsweise 157 und 163 mm liegt.

8. Knochenplatte nach Anspruch 6 oder 7, **dadurch gekennzeichnet, dass** die Länge der kleinen Achse (23) der zum Ellipsenbogen (21) gehörige Ellipse im Bereich zwischen 60 und 80 mm, vorzugsweise zwischen 67 und 73 mm liegt.

9. Knochenplatte nach einem der Ansprüche 1 bis 8, **dadurch gekennzeichnet, dass** das freie Ende (5) des stilförmigen Teils und des löffelartigen Teils (2) im wesentlichen in zueinander parallelen Ebenen liegen.

10. Knochenplatte nach einem der Ansprüche 1 bis 9, **dadurch gekennzeichnet, dass** sie mindestens in einem Teilbereich eine quer zur Zentralachse (3) verlaufende Krümmung (6) vorzugsweise mit einem Krümmungsradius im Bereich von 18 bis 22 mm, aufweist.

11. Knochenplatte nach Anspruch 10, **dadurch gekennzeichnet, dass** sie über ihre gesamte Länge eine quer zur Zentralachse (3) verlaufende Krümmung (6) aufweist.

12. Knochenplatte nach einem der Ansprüche 1 bis 11, **dadurch gekennzeichnet, dass** die freie, endständige Partie (8) des löffelartigen Teils (2) zwei symmetrisch zur Zentralachse (3) angeordneten Bohrungen (9,10) mit parallelen Bohrungsachsen aufweist, wobei die Bohrungsachsen (11,12) einen Winkel (13) von 92° - 98°, vorzugsweise von 94° - 96° mit der durch den löffelartigen Teil (2) gebildeten Fläche (14) bilden.

13. Knochenplatte nach einem der Ansprüche 1 bis 12, **dadurch gekennzeichnet, dass** die an den Übergangsbereich (26) angrenzende Partie (15) des löffelartigen Teils (2) zwei asymmetrisch zur Zentralachse (3) angeordnete Bohrungen (16, 17) aufweist.

14. Knochenplatte nach Anspruch 13, **dadurch gekennzeichnet, dass** die Bohrungsachsen (18,19) der beiden Bohrungen (16,17) in zueinander parallelen Ebenen liegen, welche vorzugsweise orthogonal zur Zentralachse (3) stehen.

15. Knochenplatte nach Anspruch 14, **dadurch gekennzeichnet, dass** die Projektion der einen Bohrungsachse (18) in die parallele Ebene, welche die andere Bohrungsachse (19) enthält, mit der Bohrungsachse (19) einen Winkel (20) von 40°-60°, vorzugsweise von 46°-54° untereinander einschliesst.

16. Knochenplatte nach einem der Ansprüche 12 bis 15, **dadurch gekennzeichnet, dass** mindestens eine der symmetrisch und/oder asymmetrisch zur Zentralachse (3) angeordneten Bohrungen (9,10,16,17) ein Innengewinde (27) oder partiell umlaufende Keilnuten aufweisen.

17. Knochenplatte nach einem der Ansprüche 12 bis 16, **dadurch gekennzeichnet, dass** sich keine der Bohrungsachsen (11,12;18,19) der symmetrisch und/oder asymmetrisch zur Zentralachse (3) angeordnete Bohrungen (9,10;16,17) schneiden.

18. Knochenplatte nach einem der Ansprüche 1 bis 17, **dadurch gekennzeichnet, dass** die Achsen der im stilförmigen Teil (1) angebrachten Bohrungen (24) senkrecht dazu stehen.

19. Knochenplatte nach einem der Ansprüche 1 bis 18, **dadurch gekennzeichnet, dass** die Innengewinde (27;28) mehrgängig, vorzugsweise zweigängig ausgebildet sind.

## Claims

1. A bone plate for the fixation of proximal humerus fractures using a handle-shaped part (1), whose length is greater than its width x, and a spoon-shaped part (2), whose width y is greater than width x of the handle-shaped part (1), the handle-shaped part (1) and the spoon-shaped part (2) having a common longitudinal axis (3) and being connected with one another via a transition area (26), and at least 2 screw holes (9,10,16,17;24) each being disposed in the handle-shaped part (1) and in the spoon-shaped part (2), whereby
at least one of the screw holes (24) in the handle-shaped part (1) has an at least partial inside thread (28) or partially circumferential grooves; and
at least one of the screw holes (9,10,16,17) in the spoon-shaped part (2) has an inside thread (27) or partially circumferential grooves,
**characterized in that**
the pitch of the inside threads (27;28) or the partially circumferential grooves lies in the range between 0,5 mm and 1,1 mm; and
the thickness (7) of the bone plate lies in the range between 1,7 and 2,3 mm.

2. The bone plate as recited in Claim 1, **characterized in that** the pitch of the inside threads (27;28) or the partially circumferential grooves lies in the range between 0,7 mm and 0,9 mm.

3. The bone plate as recited in Claim 1 or 2, **characterized in that** the width of the transition area (26) broadens, preferably exponentially, from width x of the handle-shaped part (1) to width y of the spoon-shaped part (2).

4. The bone plate as recited in one of Claims 1 through 3, **characterized in that** its thickness (7) lies in the range between 1,9 mm and 2,1 mm.

5. The bone plate as recited in one of Claims 1 through 4, **characterized in that** the handle-shaped part (1), viewed in the direction of the central axis (3), is curved in at least one partial section.

6. The bone plate as recited in one of Claims 1 through 5, **characterized in that** the handle-shaped part (1), viewed in the direction of the central axis (3), is curved in the form of a spline (21) in at least one partial section.

7. The bone plate as recited in Claim 6, **characterized in that** the length of the large axis (22) of the ellipse associated with the spline (21) is between 150 mm and 170 mm, preferably 157 mm and 163 mm.

8. The bone plate as recited in Claim 6 or 7, **characterized in that** the length of the small axis (23) of the ellipse associated with spline (21) is between 60 mm and 80 mm, preferably 67 mm and 73 mm.

9. The bone plate as recited in one of Claims 1 through 8, **characterized in that** the free ends (5) of the handle-shaped part (1) and the spoon-shaped part (2) essentially lie in planes parallel to one another.

10. The bone plate as recited in one of Claims 1 through 9, **characterized in that** it has, in at least one partial section, a curvature (6) perpendicular to the central axis (3), the curvature preferably having a curvature radius between 18 and 22 mm.

11. The bone plate as recited in Claim 10, **characterized in that** it has - over its entire length - a curvature (6) perpendicular to the central axis (3).

12. The bone plate as recited in one of Claims 1 through 11, **characterized in that** the free terminal section (8) of spoon-shaped part 2 has two holes (9,10), disposed symmetrically to the central axis (3) and having parallel hole axes, with the hole axes (11,12) forming an angle (13) of between 92° and 98°, preferably between 94° and 96°, with the surface (14) formed by the spoon-shaped part (2).

13. The bone plate as recited in one of Claims 1 through 12, **characterized in that** the section (15) of the spoon-shaped part (2), bordering the transition area (26), additionally has two holes (16,17) disposed symmetrically to central axis 3.

14. The bone plate as recited in Claim 13, **characterized in that** the hole axes (18,19) of the two holes (16,17) lie on planes parallel to one another, which are disposed orthogonally to the central axis (3).

15. The bone plate as recited in Claim 14, **characterized in that** the projection of the one hole axis (18) into the parallel plane containing the other hole axis (19) forms an angle (20) of 40°-60°, preferably 46°-54°, with the hole axis (19).

16. The bone plate as recited in one of Claims 12 through 15, **characterized in that** at least one of the holes (9,10,16,17) disposed symmetrically and/or asymmetrically to the central axis (3) have an inside thread (27) or partially circumferential grooves.

17. The bone plate as recited in one of Claims 12 through 16, **characterized in that** none of the hole axes (11,12;18,19) of the holes situated symmetrically and/or asymmetrically to the central axis (3) intersect.

18. The bone plate as recited in one of Claims 12 through 17, **characterized in that** the axes of the holes (24) in the handle-shaped part (1) are disposed perpendicularly to it.

19. The bone plate as recited in one of Claims 12 through 18, **characterized in that** the inside threads (27;28) are configured as multithreads, preferably dual-threads.

## Revendications

1. Plaque d'ostéosynthèse pour la fixation de fractures proximales de l'humérus, comprenant une partie en forme de manche (1), dont la longueur est supérieure à sa largeur x, et une partie en forme de cuiller (2), dont la largeur y est supérieure à la largeur x de la partie en forme de manche (1), dans laquelle la partie en forme de manche (1) et la partie en forme de cuiller (2) présentent un axe central longitudinal commun (3) et sont reliées l'une à l'autre par une zone de transition (26), et dans laquelle au moins deux trous de vis (9, 10, 16, 17 ; 24) sont disposés dans la partie en forme de manche (1) ainsi que dans la partie en forme de cuiller (2), dans laquelle
- au moins un des trous de vis (24) présente, dans la partie en forme de manche (1), au moins un taraudage partiel (28) ou des rainures de clavette périphériques partielles ; et
- au moins un des trous de vis (9, 10, 16, 17) présente, dans la partie en forme de cuiller (2), au moins un taraudage (27) ou des rainures de clavette périphériques partielles ;
**caractérisée en ce que**
- le pas des taraudages (27 ; 28) ou des rainures de clavette périphériques partielles est dans la gamme de 0,5 mm à 1,1 mm ; et
- l'épaisseur (7) de la plaque d'ostéosynthèse est dans la gamme de 1,7 à 2,3 mm.

2. Plaque d'ostéosynthèse selon la revendication 1, **caractérisée en ce que** le pas des taraudages (27 ; 28) ou des rainures de clavette périphériques partielles est dans la gamme de 0,7 à 0,9 mm.

3. Plaque d'ostéosynthèse selon la revendication 1 ou 2, **caractérisée en ce que** la largeur de la zone de transition (26) augmente depuis la largeur x de la partie en forme de manche (1) jusqu'à la largeur y de la partie en forme de cuiller (2), de préférence exponentiellement.

4. Plaque d'ostéosynthèse selon l'une quelconque des revendications 1 à 3, **caractérisée en ce que** son épaisseur (7) est dans la gamme de 1,9 à 2,1 mm.

5. Plaque d'ostéosynthèse selon l'une quelconque des revendications 1 à 4, **caractérisée en ce que** la partie en forme de manche (1), vue dans le sens de l'axe central (3), a une forme courbée, au moins sur un segment partiel.

6. Plaque d'ostéosynthèse selon l'une quelconque des revendications 1 à 5, **caractérisée en ce que** la partie en forme de manche (1), vue dans le sens de l'axe central (3), a la forme d'un arc elliptique (21), au moins sur un segment partiel.

7. Plaque d'ostéosynthèse selon la revendication 6, **caractérisée en ce que** la longueur du grand axe (22) de l'ellipse qui définit l'arc elliptique (21) est comprise entre 150 et 170 mm, de préférence entre 157 et 163 mm.

8. Plaque d'ostéosynthèse selon la revendication 6 ou 7, **caractérisée en ce que** la longueur du petit axe (23) de l'ellipse qui définit l'arc elliptique (21) est comprise entre 60 et 80 mm, de préférence entre 67 et 73 mm.

9. Plaque d'ostéosynthèse selon l'une quelconque des revendications 1 à 8, **caractérisée en ce que** l'extrémité libre (5) de la partie en forme de manche et celle de la partie en forme de cuiller (2) sont situées dans des plans essentiellement parallèles l'un à l'autre.

10. Plaque d'ostéosynthèse selon l'une quelconque des revendications 1 à 9, **caractérisée en ce qu'**elle présente, au moins sur un segment partiel, une courbure (6) s'étendant transversalement à l'axe central (3), de préférence avec un rayon de courbure de 18 à 22 mm.

11. Plaque d'ostéosynthèse selon la revendication 10, **caractérisée en ce qu'**elle présente, sur toute sa longueur, une courbure (6) s'étendant transversalement à l'axe central (3).

12. Plaque d'ostéosynthèse selon l'une quelconque des revendications 1 à 11, **caractérisée en ce que** la partie terminale libre (8) de la partie en forme de cuiller (2) présente deux alésages (9, 10) avec des axes d'alésage parallèles, disposés symétriquement par rapport à l'axe central (3), dans laquelle les axes d'alésage (11, 12) forment un angle (13) de 92° à 98°, de préférence de 94° à 96° avec la surface (14) formée par la partie en forme de cuiller (2).

13. Plaque d'ostéosynthèse selon l'une quelconque des revendications 1 à 12, **caractérisée en ce que** la partie (15) de la partie en forme de cuiller (2) adjacente à la zone de transition (26) présente deux alésages (16, 17) disposés asymétriquement par rapport à l'axe central (3).

14. Plaque d'ostéosynthèse selon la revendication 13, **caractérisée en ce que** les axes d'alésage (18, 19) des deux alésages (16, 17) se trouvent dans deux plans parallèles l'un à l'autre qui sont situés de préférence orthogonalement à l'axe central (3).

15. Plaque d'ostéosynthèse selon la revendication 14, **caractérisée en ce que** la projection de l'axe d'alésage (18) dans le plan parallèle, qui contient l'autre axe d'alésage (19), forme un angle (20) de 40° à 60°, de préférence de 46° à 54° avec l'axe d'alésage (19).

16. Plaque d'ostéosynthèse selon l'une quelconque des revendications 12 à 15, **caractérisée en ce qu'**au moins un des alésages (9, 10, 16, 17) disposés symétriquement et/ou asymétriquement par rapport à l'axe central (3) présente un taraudage (27) ou des rainures de clavette périphériques partielles.

17. Plaque d'ostéosynthèse selon l'une quelconque des revendications 12 à 16, **caractérisée en ce qu'**aucun des axes d'alésage (11, 12 ; 18, 19) des alésages (9, 10; 16, 17) disposés symétriquement et/ou asymétriquement par rapport à l'axe central (3) ne se coupent.

18. Plaque d'ostéosynthèse selon l'une quelconque des revendications 1 à 17, **caractérisée en ce que** les axes des alésages (24) disposés dans la partie en forme de manche (1) sont perpendiculaires à celle-ci.

19. Plaque d'ostéosynthèse selon l'une quelconque des revendications 1 à 18, **caractérisée en ce que** les taraudages (27 ; 28) sont réalisés en tant que filets multiples, de préférence en tant que filets doubles.
